# EUROPEAN PATENT APPLICATION

(11) **EP 1 528 396 A1**
(43) Date of publication of application: **04.05.2005**
(21) Application number: 04022918.9
(22) Date of filing: 27.09.2004
(51) Int. Cl.: G01N 33/569, G01N 33/50, C12Q 1/34, C12Q 1/37, G01N 33/48

(54) **Enzyme activities and pH tests for the determination of the risk of obstetric and gynecologic complications in samples of body fluids of women**

(30) Priority: 31.10.2003 US 698795
(71) Applicant: UNIBIO S.R.L., 40125 Bologna (IT)
(72) Inventor: Cauci, Sabina, 34074 Monfalcone Gorizia (IT)
(74) Representative: Siniscalco, Fabio

(57) **Abstract**

The current invention describes a method for selecting a particular population of women having a risk of developing obstetric or gynecologic pathologies indicated as odds ratio (OR) value higher than 5.5, comprising the following steps in order:
a) determination of the levels of sialidase by means of the procedure described in Cauci et al. Am J Obstet Gynecol 1998; 178; 511-5 and/or prolidase activity by means of the procedure described in Cauci et al. J Infect Dis 1998; 178; 1698-706 in samples of body fluid;
b) determination of the pH value of said body fluid samples;
c) selecting the samples having a sialidase value equal or above 5.0 nmol of methoxyphenol and/or a prolidase level equal or above 1500 mOD for prolidase and a pH ≥ 5.0.

Consequently, this method gives the physician an efficient tool to decide whether or not to administer a pharmacological therapy to women at risk of severe adverse outcomes.

## Description

In general, the present invention refers to one enzymatic test and/or two combined enzymatic tests by themselves in combination with a pH evaluation for the determination of the risk of obstetric and gynecologic complications and, in particular, to a method for the selection of a population of women having a high risk of developing pathologies correlated to obstetric and gynecologic complications, urologic disorders, sexually transmitted diseases, viral infections and malignancies in the urogenital tract.

### BACKGROUND OF THE INVENTION

Several pathologies correlated to obstetric and gynecologic complications are well known to the physician as, for example, low birth weight (<2500 g at birth, LBW), very low birth weight (<1500 g at birth, VLBW), spontaneous preterm delivery (spontaneous delivery at less than 37 weeks' gestation, PTD), early preterm delivery (delivery at less than 35, or 34, or 32 weeks' gestation, EPTD) including all preterm delivery spontaneous or induced, premature rupture of membranes (PROM), preterm premature rupture of membranes (PPROM), spontaneous abortion, early and late miscarriage, clinical or subclinical chorioamnionitis, intraamniotic infections, postpartum hemorrhage (PPH), post voluntary abortion infections, obstetric surgery infections, post-partum or post-gynecologic surgery infections, pelvic surgery infections, endometritis, upper genital tract infections which cause infertility, pelvic inflammatory disease (PID), annexitis, cervicitis, urinary infections, urinary tract disorders, sexually transmitted diseases as Chlamydia trachomatis, or Gonorrhoea, sexually acquired viral infections including human papilloma virus (HPV), herpes simplex virus (HSV), human immunodeficiency virus (HIV) and pre-malignant or malignant conditions as ASCUS and CIN (cervical intraepithelial neoplasia).

In particular, it is known that Low Birth Weight including Very Low Birth Weight, primarily due to preterm birth (PTD), is the main risk factor for neonatal morbidity, including perinatal HIV-1 transmission, newborn respiratory distress syndrome (RDS), use of mechanical ventilation for the newborn, length of infant hospital stay, maternal and newborn hospital costs, infant mortality, and long term infant neurological disorders including cerebral palsy, poor cognitive and mnemonic activities, which become evident after three or four years of age. Most cases of LBW have no recognized causal factors, making current prevention strategies ineffective. However, several investigations have shown that alterations of vaginal flora and in particular bacterial vaginosis (BV) are associated with preterm delivery (PTD) (with increased relative risk from 1.4 to 6.9 fold in respect to absence of BV), preterm delivery of low-weight infants, LBW, PROM, PPROM, abortion, early and late miscarriage, and in general with maternal complications. Anyway only a small percentage of women with BV have adverse pregnancy outcomes: 6.3% have a LBW preterm infant and 3.4% have a LBW full-term infant. Thus, more specific predictive markers for LBW and preterm delivery are needed among women with altered vaginal flora.

Bacterial vaginosis is caused by different bacterial species mainly anaerobic, or facultative but also aerobic, comprising *G*. *vaginalis, Bacteroides* spp., *Prevotella* spp. and *Mobiluncus* spp., gram positive cocci, *Ureaplasma urealyticum* and *Mycoplasma hominis.* The pathogenesis of bacterial vaginosis is still obscure and not always the presence of such pathologic condition is easy to diagnose. In particular, it is not known what triggers the shift from the normal lactobacilli colonization to the altered vaginal flora, or which degree of the vaginal microbial flora alteration is indicative of a real pathologic condition.

A crucial issue in bacterial vaginosis is the synergy between *G*. *vaginalis* and anaerobic bacteria. *G*. *vaginalis* is found in high titers in over the 95% of bacterial vaginosis cases, whereas low titers of G. *vaginalis* colonization are found also in women without bacterial vaginosis. However, a strict correlation between *G*. *vaginalis* colonization and the condition of bacterial vaginosis has been demonstrated. The only fully characterized virulence factor released by *G*. *vaginalis* is a β-hemolytic toxin (Gvh) having a molecular weight of about 59,000 Dalton. Furthermore, it has been shown that about half of the non-pregnant women develop an immune response against Gvh eliciting IgA antibodies (anti-Gvh IgA) at the vaginal mucosa level. The absence of anti-Gvh IgA antibodies in non-pregnant women with BV is mainly due to the inactivation of IgA and this phenomenon is related to the presence of high levels of vaginal hydrolytic enzyme activities, specifically sialidase and prolidase activities. Such inactivation of vaginal immunoglobulins derives from a structural impairment of IgA likely due to the hydrolytic action of an array of factors released by anaerobic bacteria.

. Presently it is not known how many and which factors of the antimicrobial host defense are compromised or which microbial virulence factor(s) elicits the IgA cleavage in a subgroup of women with bacterial vaginosis.

However it is demonstrated that elevated vaginal IgA levels are protective for LBW and PTD.

The uncertain definition of the kind of alteration of the vaginal flora which is a real pathologic condition makes clinicians to face the problem whether or not administer a pharmacological therapy to women with a certain grade of altered vaginal flora or with a diagnosis of bacterial vaginosis. It is well known that two different drugs are used to treat bacterial vaginosis: metronidazole or clindamycin. Both these drugs show a main withdraw consisting in relevant adverse side effects, in particular metronidazole can give gastrointestinal symptoms, and is considered a teratogen agent, so it is not recommended during pregnancy, on the other hand clindamycin can cause diarrhea and pseudomembranous colitis which may be lethal. Moreover both such antibiotics can cause yeast vaginitis.

The prevalence of bacterial vaginosis on the overall female population is estimated around 10-20% in Western Europe; 10-50% in America, with black and Hispanic population having the higher frequencies; 30-60% in Africa; 20-50% in Asia. Not all such BV positive women can be pharmacologically treated for BV because of possible side effects of treatments and/or because of costs, thus more predictive markers to select pregnant and non-pregnant, fertile, peri-menopausal and post-menopausal women really at risk of adverse complications are needed.

For the above described reasons it is highly desirable the development of a method to select a population of women having an high risk of pathologic complications, as for example those identified above.

. In addition, the determination of said population should identify women who may have an high risk of complications especially in the earliest stage of pregnancy or, advantageously, before pregnancy so that the physician can be put into the best condition to decide whether and when to submit the above treatment to the patient. Consequently, the relevant side-effects can be advantageously avoided.

. WO 02/065122 of the same applicant discloses an enzymatic test for the determination of the risk of pathologies related to the presence of sialidase and prolidase activity in women body fluid samples. In particular, said test consists in the steps of a) determining the levels of sialidase and/or prolidase activity in said sample, b) comparing said levels with ranges of prefixed values and c) calculating the risk factor. Further, the risk of pathologies is calculated considering those samples of vaginal fluid having a pH equal or higher than 4.7.

It is to be noticed that said test is not useful to select a particular population of women having a determined risk but it is in general directed to determine the risk.

. In addition, the test can not discriminate or identify women which can develop the above pathologies, as early preterm birth, in an early gestation period.

### SUMMARY OF THE INVENTION

A first object of the present invention is to solve the problem of providing a reliable method to identify a population of women having a high risk of developing the above complications in order to furnish the physician with a valuable tool to decide whether or not to administer a pharmacological therapy.

A second object of the invention is to solve the problem of providing a method to identify a population of women having a high risk of developing the above complications at an early stage of gestation.

. Such problems are solved by a method for the selection of a population of women having a high risk of pathologies correlated with sialidase and/or prolidase and pH levels in body fluid samples as expressed in the main enclosed claim.

. Further objects and advantages of the method of the present invention will be highlighted by the subsequent description of preferred embodiments, given at indicative and not limitative title.

### DETAILED DESCRIPTION OF THE INVENTION

By means of lots of detailed studies, it has been surprisingly found that the identification of a threshold of pH ≥ 5.0 in combination with a high sialidase and/or prolidase activity in body fluid samples is a crucial issue to select women who have a risk of developing the above pathologies which is dramatically 20-folds or even 30-folds higher than normal women.

. It can be understood that according to the present invention it is possible to determine those women who have not only a risk of developing the pathologies but advantageously also those women who have such an high risk as it has never been identified before and in addition it is possible to determine the risk for the worst adverse outcomes at a very early stage of gestation.

. It is to be noticed that said method is applicable to both pregnant and non-pregnant, fertile, peri-menopausal and post-menopausal women.

For the setting up of the method of the current invention clinical cases have been studied and compared with controls of women with no recognized pathologic conditions. In particular, sialidase activity was measured in 133 women who had low birth weight babies (LBW, <2500g), among them 24 women who had very low birth weight babies (VLBW, <1500g), and 467 women who had normal birth weight babies at term of gestation (NTD, newborn infant weighting >2500 g). Prolidase activity was measured in 131 women who had low birth weight babies (LBW), among them 23 women who had very low birth weight babies (VLBW), and 465 women who had normal birth weight babies at term of gestation (NTD, newborn infant weighting >2500 g).

The presence of sialidase and/or prolidase activity was detected in the vaginal fluid samples of all these women, moreover the levels of said sialidase and prolidase activity were evaluated. Also pH levels were detected.

Sialidases are enzymes produced by bacteria and are involved in the pathogenesis of several diseases. These enzymes provoke the hydrolysis of sialic acid from various glycoproteins including IgA, and innate immunity factors as interleukins, lactoferrin, SLPI, thus altering the immune response. Prolidases are proteolytic enzymes produced by bacteria to favor the cell infiltration. These enzymes are able to activate cytokines and other immune mediators.

The sialidase activity was determined by incubation of 50 µl of the vaginal fluid sample with 50 µl of a substrate at pH 5.0, with a procedure described by Cauci et al., Am J Obstet Gynecol 1998; 178: 511-5. Specific activity was expressed as nanomoles of methoxyphenol produced from conversion of the substrate and calculated by comparison with a standard curve of pure methoxyphenol. The levels of sialidase activity were evaluated as: no activity for values below 0.19 nmol of methoxyphenol, +1 cutoff for values equal or above 0.19 nmol of methoxyphenol; +2 cutoff for values equal or above 0.38 nmol of methoxyphenol; +3 cutoff for values equal or above 2.50 nmol of methoxyphenol, and +4 cutoff for values equal or above 5.0 nmol of methoxyphenol.

Prolidase activity was determined as described by Cauci et al., J Infect Dis 1998; 178:1698-706. The levels of prolidase activity were evaluated as: no activity for values below 22 mOD, +1 cutoff for values equal or above 22 mOD; +2 cutoff for values equal or above 44 mOD; +3 cutoff for values equal or above 1000 mOD; +4 cutoff for values equal or above 1500 mOD and +5 cutoff for values equal or above 2000 mOD.

The +1 cutoffs of sialidase and prolidase activity were calculated by the mean value plus one standard deviation measured in a healthy control population (that is devoid of any vaginal symptom and with no microbial vaginal colonization except lactobacilli). The +2 cutoff was obtained by doubling the +1 cutoff. For sialidase activity the +3 cutoff was obtained by halving the +4 cutoff. For prolidase activity the +3 cutoff was obtained by halving the +5 cutoff. For prolidase activity the +4 cutoff was half the way from the +3 and the +5 cutoff.

The enclosed Table 1 shows data deriving from the correlation between the vaginal marker pH ≥ 5.0 alone or in combination with the sialidase levels (≥+1 to ≥+4) or prolidase levels (≥+1, ≥+3, ≥+4 and ≥+5) and newborn infant weighting more than 2500 g (NBW), LBW or VLBW. In particular, on the first line, from left to right, the data show the percentage % of NBW women who have had a pH ≥ 5.0, the percentage of LBW women who have had a pH ≥ 5.0, the p-value, the odds ratio (OR) and the 95% confidence interval (CI) value. Further, continuing on the same line, the data show the percentage % of VLBW women who have had a pH ≥ 5.0, the p-value, the odds ratio and the 95% CI value. On the second line, from left to right, the data show the percentage % of women with both a pH ≥ 5.0 and a sialidase level ≥+1 following the above criteria. Similarly, on the remaining lines, the same criteria has been repeated for the other sialidase and prolidase levels above specified.

. It is to be noticed that the data in bold show the risk coefficient (odds ratio, OR) derived from the ratio between the percentages of women with LBW or VLBW and the percentage of NBW women, respectively. This statistical approach was chosen to evaluate the risk of the outcome as it is the generally accepted method to estimate the relative risk in retrospective case-control studies as the ones of the current paper ("Practical Statistics for Medical Research", Douglas G. Altman, Chapman and Hall Editor, London). In particular, odds ratios were calculated to estimate the risk for each adverse pregnancy outcome. The odds ratios can be calculated by means of known statistical programs such as, for instance, SPSS computer statistic program.

Unvaried comparisons of proportions are also reported and were carried out using X²-test (Pearson two-tailed test, in the table p-value). Any p-value of < 0.05 was considered statistically significant.

Finally, 95% confidence intervals (CIs) were calculated to assess confidence ranges of odds ratios, i.e. a range of values which we can be confident has the 95% probability to include the true value, statistical significant 95% CI ranges of OR were those with a lower limit of 1.0.

In particular, the vaginal fluid samples were obtained from women in the first or in the second trimester of gestational age, or in the third trimester with intact membranes and, preferably, in the period ranging from the sixth to the twenty-fourth full week of gestation.

. The main result shown in Table 1 is the OR value as it represents the index of risk of low birth weight (LBW) or very low birth weight (VLBW).

. If this ratio is lower than 1 it means that the woman has no risk of complications or she is protected against the adverse outcome. A ratio ranging from 1 to 2 is associated with a medium risk of complications. If this ratio is comprised between 2 and 5.5 it means that the woman has a high risk of complications. Finally, if this ratio is higher than 5.5 it means that the woman has an extremely high risk of pregnancy adverse outcomes as low birth weight or preterm birth.

. In particular, and at title of example, a medium risk corresponding to an OR value equal 2 means that the patient has a twofold risk of complications compared to a normal woman, that is, in other words, a risk 100% higher.

Preferably, this result can be point out with a score: - meaning a low or no risk, + meaning a medium risk, ++ meaning a high risk, and +++ meaning an extremely high risk.

Table 1 highlights that, for LBW, a pH ≥ 5.0 alone corresponds to a not significant OR of 1.6 (p-Value of 0.050, 95% CI of 0.99-2.4). When a pH ≥ 5.0 is detected in combination with sialidase activity according to cut off ≥+1 to ≥+3, the respective significant ORs are 3.5, 3.3 and 3.8. However, when pH ≥ 5.0 is combined with sialidase activity ≥+4, the OR is surprisingly 9.9, i.e. about 3-fold higher than the other ORs.

The same trend can be observed for VLBW, wherein, in particular, the OR correlated to a pH ≥ 5.0, combined with sialidase activity ≥+4, is dramatically high (22).

. The second half of Table 1 shows data related to the prolidase levels. The same observation made with reference to the sialidase levels can be done for prolidase levels. In particular, for LBW the OR surprisingly increases more that 3-folds when a pH ≥ 5.0 is detected in combination with very high prolidase levels of ≥+5 (OR = 9.2, 95% CI 1.8-48 and p = 0.001) with respect to the ORs resulting from the combination of pH ≥ 5.0 and prolidase levels of ≥+1 and ≥+3.

Further, unexpectedly, very high ORs for VLBW are observed in women with very high prolidase (≥+5 cut off) and pH ≥ 5.0 (OR = 22, 95% CI 3.0-164, p < 0.001).

. In other words, from the above data, it comes out that there is a substantial increment in the OR values corresponding to an increment in sialidase levels in the presence of a pH ≥ 5.0. However, it has been found a dramatic increment in the OR values when a high sialidase level and a pH ≥ 5.0 are detected. This means that when sialidase levels of ≥+4 and a pH ≥ 5.0 are detected there exists a very high risk of LBW as well as of VLBW.

Likewise, there is a substantial increment in the OR values corresponding to an increment in prolidase levels, however a dramatic increment in the OR values has been detected when there are high (≥+4 or ≥+5) prolidase level and a pH ≥ 5.0. This means that when prolidase levels ≥+4 or ≥+5 and a pH ≥ 5.0 are detected there exists a very high risk of LBW as well as of VLBW.

In a second stage of the above clinical studies, the same approach disclosed with reference to the above Table 1 has been followed in order to calculate the risk of LBW and VLBW babies in connection with a pH ≥ 5.0 combined with prolidase and sialidase levels. In particular, with reference to Table 2, for each of the four sialidase levels increasing prolidase levels have been associated. What appears from the data is that a combination between pH ≥ 5.0, any one of the sialidase levels ≥+1, ≥+2 or ≥+3 and prolidase level ≥+4 or ≥+5 results in a significant OR equal or above 5.5 for LBW as well as for VLBW. In addition, a combination of pH ≥ 5.0, the sialidase level ≥+4 and any one of prolidase levels ≥+1, ≥+3, ≥+4 or ≥+5 results in a dramatic OR well above 5.5 for LBW as well as for VLBW (see last group of data in Table 2).

. In a third stage of the above studies, the same approach disclosed with reference to Table 1 has been followed in order to calculate the risk of the spontaneous delivery (non medically indicated), including initiation with both rupture of membranes and labor before 37 weeks' gestation (PTD). In particular, sialidase activity has been measured on 418 NTD women and 106 PTD women. Prolidase activity was measured on 416 NTD women and 104 PTD women.

Table 3 shows data deriving from the correlation between vaginal pH ≥ 5.0 level alone or in combination with sialidase (≥+1 to ≥+4) or prolidase levels (≥+1 to ≥+5) and newborn infant weighting more than 2500 g (NTD) or PTD. All the parameters analyzed corresponds to those of Table 1. In particular, Table 3 shows that a pH ≥ 5.0 alone is not statistically associated with any preterm delivery at < 37 weeks' gestation. However, a pH ≥ 5.0 associated with sialidase levels from ≥+1 to ≥+3 results in a significant increasing OR respectively 3.4, 3.8 and 4.7. Surprisingly, a pH ≥ 5.0 associated with a sialidase level of ≥+4 shows a dramatic OR of 34 (p-Value<0.001, 95% CI 4.2-275). Likewise, a pH ≥ 5.0 associated with prolidase levels from ≥+1 to ≥+3 results in statistically significant increasing ORs respectively 2.2, 2.9 and 4.8. Surprisingly, a pH ≥ 5.0 associated with a prolidase level of ≥+4 shows a OR of 6.9 and a dramatically large OR of 17 (p-Value<0.001, 95% CI 4.2-275) when the prolidase level is ≥+5.

In a further stage of the above clinical studies, the same approach disclosed with reference to Table 2 has been followed in order to determine the risk of PTD associated with a pH ≥ 5.0 combined with prolidase and sialidase levels. In particular, with reference to Table 4, for each of the four sialidase levels increasing prolidase levels have been associated. What appears from the data is that a combination between pH ≥ 5.0, any one of the sialidase levels ≥+1, ≥+2 or ≥+3 and prolidase levels ≥+4 or ≥+5 results in significant ORs above 5.5 for PTD. In addition, a combination of pH ≥ 5.0, the sialidase level ≥+4 and prolidase levels ≥+1 or ≥+2 results in a extremely high ORs well above 5.5 for PTD (see last group of data in Table 4). It is also to be noticed that no countable data are found for pH ≥ 5.0, sialidase level of ≥+3 and prolidase level of ≥+5 and for pH ≥ 5.0, sialidase level of ≥+4 and prolidase levels of ≥+3, ≥+4 or ≥+5 as none NTD was found to have said values in combination. In other words, it means that those patients who have said values have had PTD.

In a further phase of the above clinical studies, the same approach disclosed with reference to Table 1 has been followed in order to calculate the risk of all the delivery at less than 37 weeks' gestation (<37 wks), 35 weeks' gestation (<35 wks) and at less than 32 weeks' gestation (<32 wks). In particular, sialidase activity has been measured on 418 NTD women, 138 women who have had a delivery at less than 37 weeks' gestation, 57 who have had a delivery at less than 35 weeks' gestation and 22 women who have had a delivery at less than 32 weeks' gestation. Prolidase activity was measured on 416 NTD women, 136 who have had a delivery at less than 37 weeks' gestation, 55 women who have had a delivery at less than 35 weeks' gestation and 21 women who have had a delivery at less than 32 weeks' gestation.

Table 5 shows that a pH ≥ 5.0 alone has a not statistically significant OR of 1.3. While, when a pH ≥ 5.0 is associated to sialidase levels of ≥+1, ≥+2, ≥+3 significant increasing ORs are found for delivery at less than 37 weeks' gestation (2.9, 3.1, 3.5). Similar results have been found for delivery at less than 35 weeks' gestation (3.3, 3.5, 4.8 respectively). A higher increase in ORs can be observed for delivery at less than 32 weeks' gestation (5.2, 6.2, 10 respectively). Remarkably the combination of high sialidase (≥+4) and pH ≥ 5.0 has statistically significant impressively large ORs, specifically OR of 26 for delivery at less than 37 weeks' gestation, of 40 for preterm at < 35 weeks' gestation and of 66 for delivery at less than 32 weeks' gestation.

The same trend can be observed for prolidase levels of ≥+1, ≥+2 or ≥+3 for delivery at less than 37 weeks' gestation, at less than 35 weeks' gestation and less than 32 weeks' gestation. In particular, prolidase +4 in combination with pH ≥ 5.0 has OR of 5.8 for delivery at less than 37 weeks' gestation, of 8.2 for preterm at < 35 weeks' gestation and of 19 for preterm at < 32 weeks' gestation. Prolidase levels of ≥+5 in combination with pH ≥ 5.0 has a dramatic OR of 16 for both preterm at less than 37 weeks' gestation and < 35 weeks' gestation and of 44 for preterm at < 32 weeks' gestation.

. In a still further phase of the above clinical studies, the same approach disclosed with reference to Table 2 has been followed in order to determine the risk of delivery at < 37 weeks' gestation, at < 35 weeks' gestation and < 32 weeks' gestation associated with a pH ≥ 5.0 combined with prolidase and sialidase levels. In particular, with reference to Table 6, for each of the four sialidase levels increasing prolidase levels have been associated. What appears from the data is that a combination between pH ≥ 5.0, any one of the sialidase levels ≥+1, ≥+2 or ≥+3 and prolidase levels ≥+4 or ≥+5 results in a significant OR above 5.5 for all the considered cases. In addition, a combination of pH ≥ 5.0, the sialidase level ≥+4 and prolidase levels ≥+1 or ≥+2 results in dramatically high ORs well above 5.5 for all the considered cases (see last group of data in Table 6). It is also to be noticed that no countable data are found for pH ≥ 5.0, sialidase level of ≥+3 and prolidase level of ≥+5 and for pH ≥ 5.0, sialidase level of ≥+4 and prolidase levels of ≥+3, ≥+4 or ≥+5 as none delivery at <37 weeks' gestation, at < 35 weeks' gestation or < 32 weeks' gestation was found to have said values in combination. In other words, it means that all those patients who have said values have had delivery at < 37 weeks' gestation, or < 35 weeks' gestation or < 32 weeks' gestation, respectively.

From the analysis of the clinical data shown in the above commented Tables, it is possible to relate the high risk for LBW, VLBW, PTD, delivery at < 37 weeks' gestation, or < 35 weeks' gestation or < 32 weeks' gestation, respectively with the levels of sialidase and/or prolidase activity and pH threshold of ≥ 5.0 in the body fluid of the patient.

. On the grounds of the results just shown, and in agreement with the present invention, a method has been set up for selecting a particular population of women having a risk of developing obstetric or gynecologic pathologies indicated as risk coefficient OR value equal or higher than 5.5, comprising the following steps in order:
a) determination of the levels of sialidase by means of the procedure described in Cauci et al. Am J Obstet Gynecol 1998; 178; 511-5 and/or prolidase activity by means of the procedure described in Cauci et al. J Infect Dis 1998; 178; 1698-706 in a sample of body fluid;
b) determination of the pH value of said body fluid sample;
c) selecting the samples having a sialidase value equal or above 5.0 nmol of methoxyphenol and/or a prolidase level equal or above 1500 mOD for prolidase and a pH ≥ 5.0.

In particular, the body fluid sample can be for example a blood sample or, preferably, a vaginal fluid sample.

The step c) can be carried out by means of any of the known method such as a pHmeter or a reactive strip. In particular, a pH threshold of pH ≥ 5.0 and ≤ 7.0 is detected, preferably pH is ≥ 5 and ≤ 6, more preferably ≥ 5.0 and ≤ 5.5. Moreover, the prolidase level is preferably equal or above 2000 mOD.

. It is to be noticed that the method of the invention is not only useful to select that population of women having a high risk of developing the above pathologies equal or higher than 5.5 OR but also to identified particular sub-population having a risk calculated as OR between 6.0 and 66, advantageously between 9.0 and 44, more advantageously between 12 and 20. In particular, for LBW said value is comprised between 5.5 and 18, for VLBW it is comprised between 7 and 42, for PTD between 6 and 34, for delivery at less than 37 weeks' gestation between 5.5 and 26, for delivery at less than 35 weeks' gestation between 7.0 and 40 and delivery at less than 32 weeks' gestation between 10 and 66.

In view of said data, according to another object of the present invention, it is provided a method for selecting a population of women having the above risk of complication at a time less than 37 weeks' gestation, or preferably less than 35 weeks' gestation or more preferably less than 32 weeks' gestation.

. Moreover, with reference to Table 1, it is to be noticed that for VLBW ORs values above 5.5 are found also when a pH ≥ 5.0 and a sialidase or prolidase level of ≥+3 are detected. In addition, from Table 2, ORs over 5.5 are found when a pH ≥ 5.0 and a prolidase level of ≥+3 and sialidase level of ≥+1 or ≥+2 or ≥+3 are detected for VLBW.

. From Table 4, an OR of 6.2 is associated with the presence of pH ≥ 5.0, sialidase level of ≥+3 and prolidase level ≥+3 for PTD.

On Table 5, for delivery at < 32 weeks' gestation ORs over 5.5 are associated with the presence of pH ≥ 5.0 and a prolidase level of ≥+2 or sialidase level ≥+2.

On Table 6, for delivery at < 35 and < 32 weeks' gestation ORs over 5.5 are associated with the presence of pH ≥ 5.0 and a prolidase level of ≥+3 and sialidase level of ≥+1 or ≥+2 or ≥+3, as for VLBW.

Therefore, according to another object of the invention, the method for selecting a particular population of women having a risk of developing VLBW, delivery at < 37 weeks' gestation, < 35 weeks' gestation or < 32 weeks' gestation, comprising the following steps in order:
a) determination of the levels of sialidase by means of the procedure described in Cauci et al. Am J Obstet Gynecol 1998; 178; 511-5 and/or prolidase activity by means of the procedure described in Cauci et al. J Infect Dis 1998; 178; 1698-706 in a sample of body fluid;
b) determination of the pH value of said body fluid sample;
c) selecting the samples having a pH ≥ 5.0 and a sialidase value above 0.19 nmol of methoxyphenol and/or a prolidase value above 22 mOD.

Preferably, said method is carried out for selecting the above population wherein the risk is indicated as OR value equal or higher than 5.5, which value is calculated as generally accepted to estimate the relative risk in case-control studies, preferably by the commercial SPSS computer statistic program. Further, the same particular OR ranges above identified can be detected.

According to a further object of the invention, the step c) comprises selecting the samples having a pH ≥ 5.0, sialidase level of over 2.50 nmol of methoxyphenol or prolidase level of over 1000 mOD.

According to a further object, the step c) comprises selecting the samples having a pH ≥ 5.0, a sialidase value above 0.19 nmol or 0.38 nmol or 2.5 nmol of methoxyphenol when it is selected a prolidase value of over 1000 mOD.

According to a still further object, the step c) comprises selecting the samples having a pH ≥ 5.0, a sialidase value of 0.38 nmol of methoxyphenol and a prolidase value of over 22 mOD or over 44 mOD or over 1000 mOD or over 1500 mOD or over 2000 mOD.

. It is now evident which are the advantages with respect to the method according to the present invention.

First of all, said method allows to select a particular population of women having a risk of developing obstetric or gynecologic pathologies indicated as OR value higher than 5.5 calculated as disclosed above. Therefore, a very important selection among women who can develop said pathologies can be put at the attention of the physician. On the contrary, the above identified prior method can not make such a critical selection.

Secondly, the method is able to predict if the risk is within the 37 weeks' gestation or within 35 or even within 32 weeks' gestation. Thirdly the method is able to predict the risk of birth of an infant of less than 1500 g which is associated with severe morbidity and high rate of newborn death. These further data are to be considered very crucial ones because they give the physician a tool to individuate patients at risk of highly severe adverse outcomes by evaluating clinical status of a patient very early so that a therapy can be conducted in the best conditions for both the mother and the baby.

. In addition, as reported above, the present invention allows to predict the very high risk even form non-pregnant women just by detecting the sialidase and/or prolidase activity and pH value according to the above method. It can be understood that in said last case the method becomes incredibly important because it can give to the physician fundamental clinical data to set up a due therapy.

. From here on, an example of determination of the sialidase and prolidase activity in a sample of vaginal fluid will be reported at indicative and not limitative title of the current invention.

### DETERMINATION OF SIALIDASE ACTIVITY

50 *µ*l of vaginal fluid sample are incubated for 90 min at room temperature with 50 *µ*l of 2-(3'-methoxyphenyl)-N-acetyl-D-neuraminic acid (Sigma) substrate dissolved in sodium acetate 50 mM at pH = 5. Samples with sialidase activity develop a red color measurable at 492 nanometers, after addition of a developing solution of 4 mM 4-aminoantipyrine and 6 mM potassium ferricyanide. Results are expressed as difference between the absorbance of the sample after addition of the developing solution and that of a duplicate sample to which the developing reagents are not added. Specific activity was expressed by comparison with a standard curve of pure methoxyphenol.

Alternatively, the sialidase activity assay can be performed with chromogenic or fluorogenic substrates different from 2-(3'-methoxyphenyl)-N-acetyl-D-neuraminic acid (Sigma, M1393), as 2-O-(o-nitrophenyl) -alfa-D-N-acetyl neuraminic acid (Sigma, N1266, N1516), 2'-(4-methylumbelliferyl)-alfa-D-N-acetyl neuraminic acid sodium salt (Sigma, M8639), 5-bromo-4-chloro-3-indolyl-alfa-D-N-acetyl neuraminic acid (X-NeuNAc), (Sigma, B 4666; or Calbiochem 203770).

### DETERMINATION OF PROLIDASE ACTIVITY

50 *µ*l of vaginal fluid sample are incubated for 24 hours at 37°C with 50 *µ*l of L-proline-para-nitroanilide substrate (Sigma) dissolved in 100 mM sodium acetate at pH = 5. Samples with prolidase activity develop a yellow color measured at 405 nanometers. A duplicate sample without the substrate is used to subtract the sample background, whereas the spontaneous hydrolysis of the substrate is subtracted by the average value of two blanks without the vaginal fluid. Results are expressed as milli optical density units (mOD).

. Alternatively, the assay of prolidase activity can be performed with chromogenic or fluorogenic substrates different from L-proline-para-nitroanilide (Sigma P5267) as: L-proline-beta-naphthylamide (Sigma P1380), N-benzyloxycarbonyl-L-prolyl-beta-naphthylamide, N-benzyloxycarbonyl-L-proline-para-nitrophenyl ester (Sigma C4877), hydroxy-L-prolyl-beta-naphthylamide, L-proline-7-amido-4-methyl-coumarin (Sigma P5898), L-proline-4-methoxy-beta-naphthylamide (Sigma P9655).

### DETERMINATION OF pH VALUES

PH values of vaginal fluid were obtained by paper strips (Merck, range pH 4.0-7.0).

A further object of the present invention is a kit for the determination of women having a risk of developing obstetric or gynecologic pathologies indicated as OR value higher than 5.5, calculated as described above, comprising a sialidase and/or prolidase activity assay in solution that includes a colorless substrate solution in which to inoculate the biologic sample; a developing solution in a container equipped with dispenser; a reference scale to correlate the level of the sialidase activity equal or above 0.19 nmol, 0.38 nmol, 2.50 nmol or 5.0 of methoxyphenol and/or prolidase activity equal or above 22 mOD, 44 mOD, 1000 mOD, 1500 mOD or 2000 mOD with the intensity of the developed color; a pH indicator; a reference scale to correlate the pH detected by said indicator with a pH ≥ 5.0 and an illustrative leaflet containing the instructions for the proper use of the kit.

According to a further embodiment, said kit can also comprise a sialidase and/or prolidase activity test on solid support, as a platform, comprised of two membranes supported on a solid frame, and containing an enzyme substrate and an agent for the development of the color, through which the biological sample is passed; a reference scale to evaluate the entity of the enzymatic activity by the intensity of the developed color; a pH indicator; a reference scale to evaluate the pH value and an illustrative leaflet containing the instructions for the proper use of the kit.

According to a further embodiment, said kit can also comprise a sialidase and/or prolidase activity test on a solid support, comprising a membrane impregnated with a chromogenic or fluorogenic substrate of the enzyme and eventually an agent for the color development, supported on an inert strip, to be touched with the biological sample. Said kit will be furnished with a reference scale to evaluate the entity of the enzymatic activity by the intensity of the developed color.

The pH indicator is preferably a pH revealing paper with a turning interval in the range between 5.0 and 7.0, preferably between 5.0 and 6.0, more preferably between 5.0 and 5.5.

The reference scale for the enzymatic activity can report standard values calculated for example with the above identified procedures and indicated with the sings +1, +2, +3, and +4. Said standard values are associated with for example the enzyme detecting colors.

. Similarly, the reference scale for the pH values can be reported for example as less than 5.0, comprised between 5.0 and 7.0 or 5.0 and 6.0 or 5.0 and 5.5 and associated with a particular color or intensity of the same color.

The illustrative leaflet should indicate the relationship between the enzymatic activity and the pH value detected in the body fluid sample. Therefore, if for example the enzymatic activity is detected as +4 for sialidase and +1 for prolidase and the pH is between 5.0 and 5.5 the illustrative leaflet should indicate the presence of a risk equal or higher than 5.5 OR.

. It is now evident that, with reference to the evaluations contained in the above shown Tables and to the evaluations obtained from the described or anyhow available analytical tests, a kit that can provide in a fast way the determination of the risk of the above described pathologies is of major value to furnish the physician effective tools to decide about the pharmacological cure to administer to patients and to verify the efficacy of therapy.

. As can be appreciated from what described, the method to identify a population of women having a high risk of developing pathologies correlated to obstetric and gynecologic complications as, for example, low birth weight (LBW), very low birth weight (VLBW, preterm delivery (PTD), early preterm delivery (EPTD, preterm rupture of membranes, intraamniotic infections, spontaneous abortion, endometritis, obstetric surgery infections, post-partum or post-gynecologic surgery infections, pelvic surgery infections, upper genital tract infections which cause infertility, pelvic inflammatory disease (PID), annexitis, cervicitis, urinary infections, sexually transmitted diseases and viral infections allows to overcome the above mentioned problems.

Obviously an expert operator in the field, to satisfy contingent and specific demands, could introduce several modifications and embodiments to the above described method, but all these are contained in the invention as defined by the following claims.

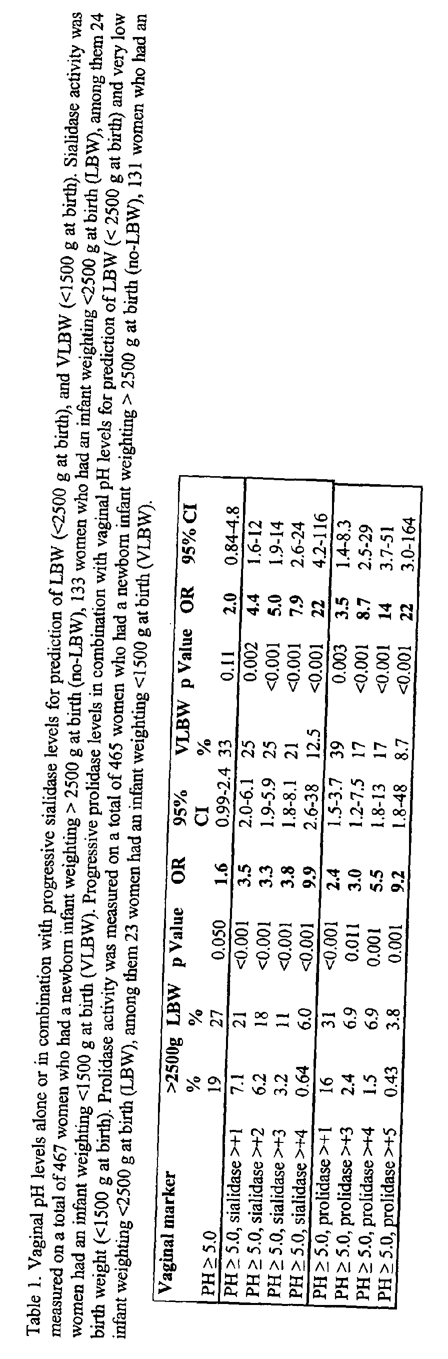

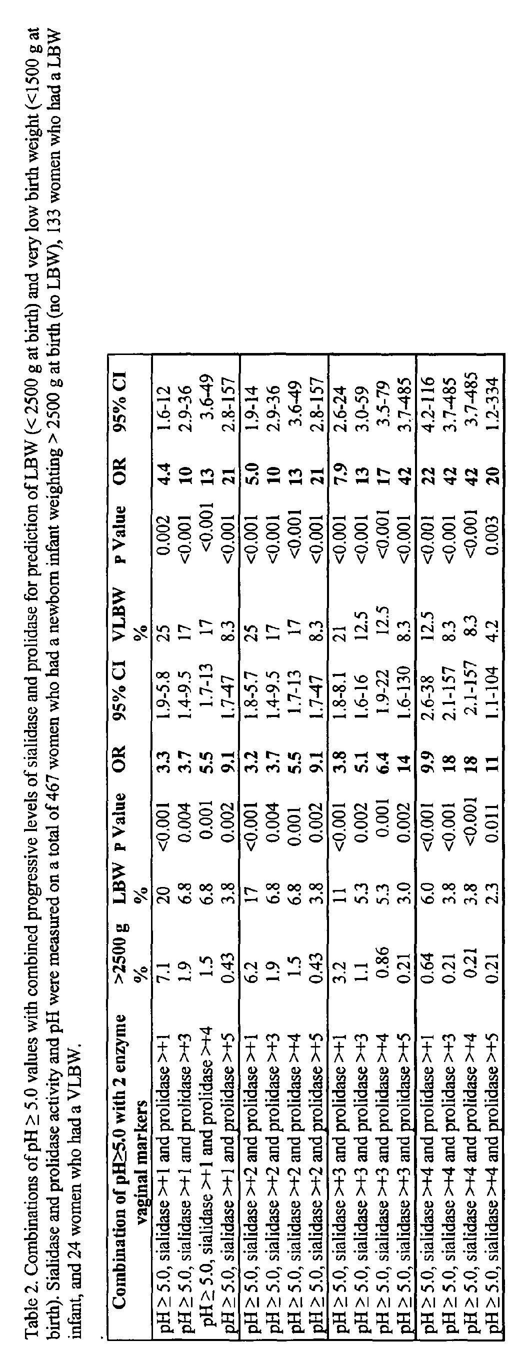

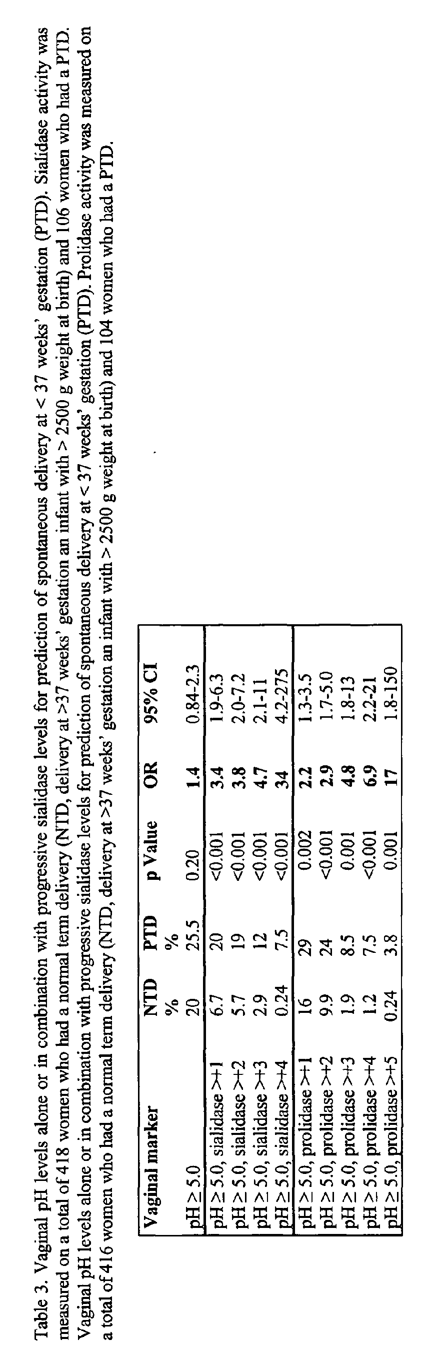

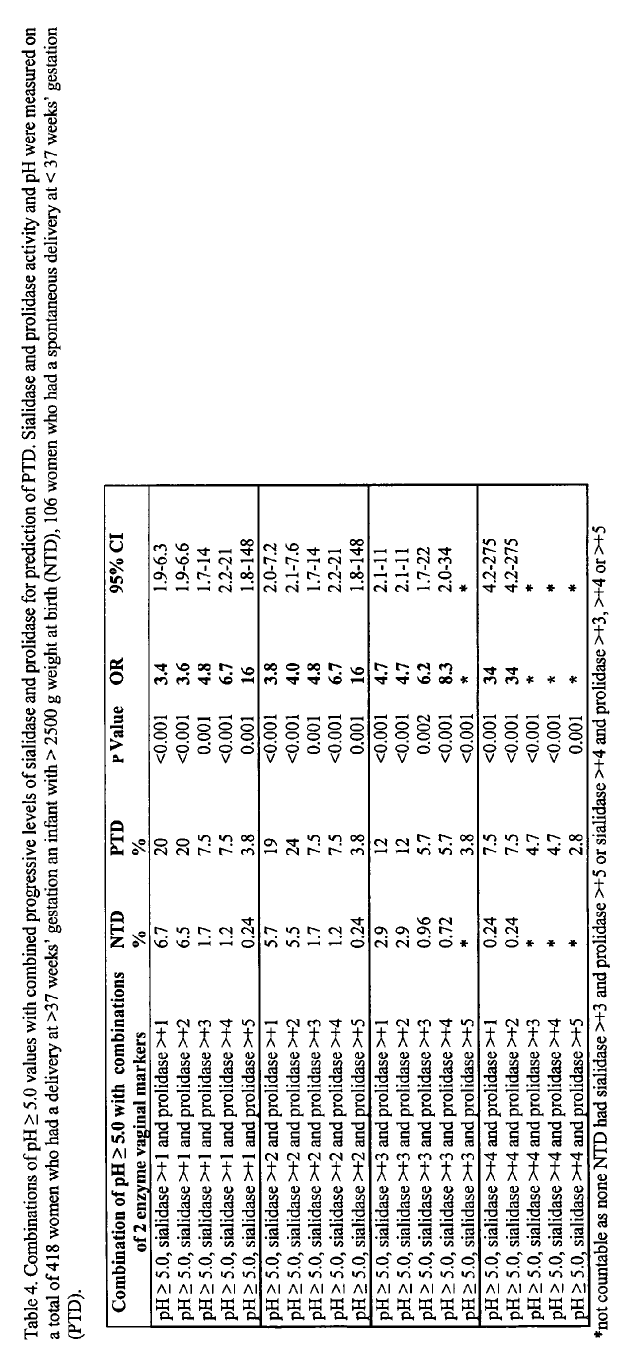

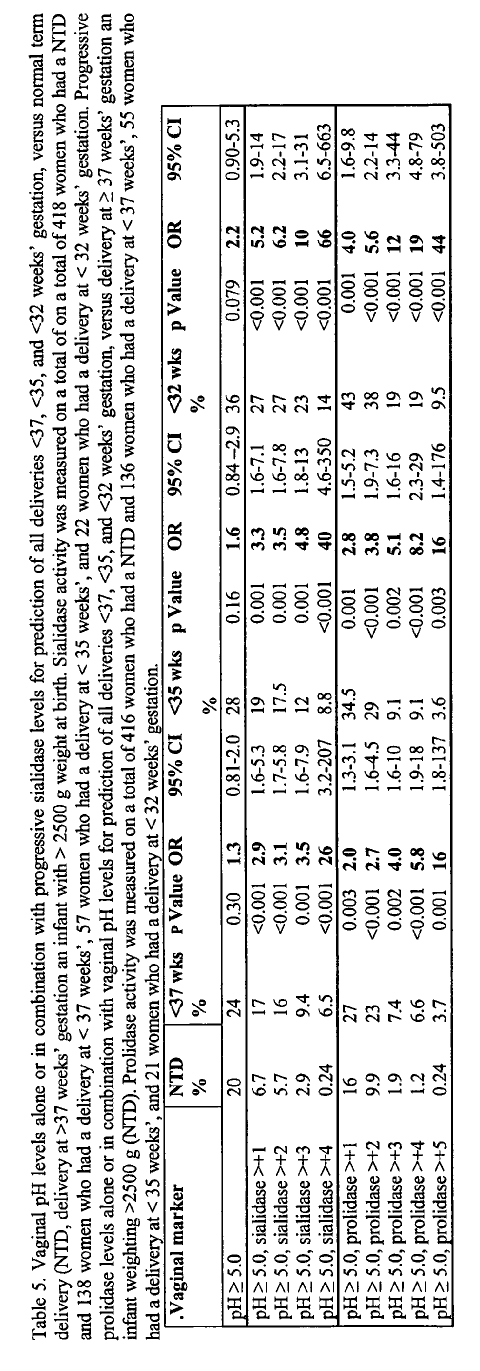

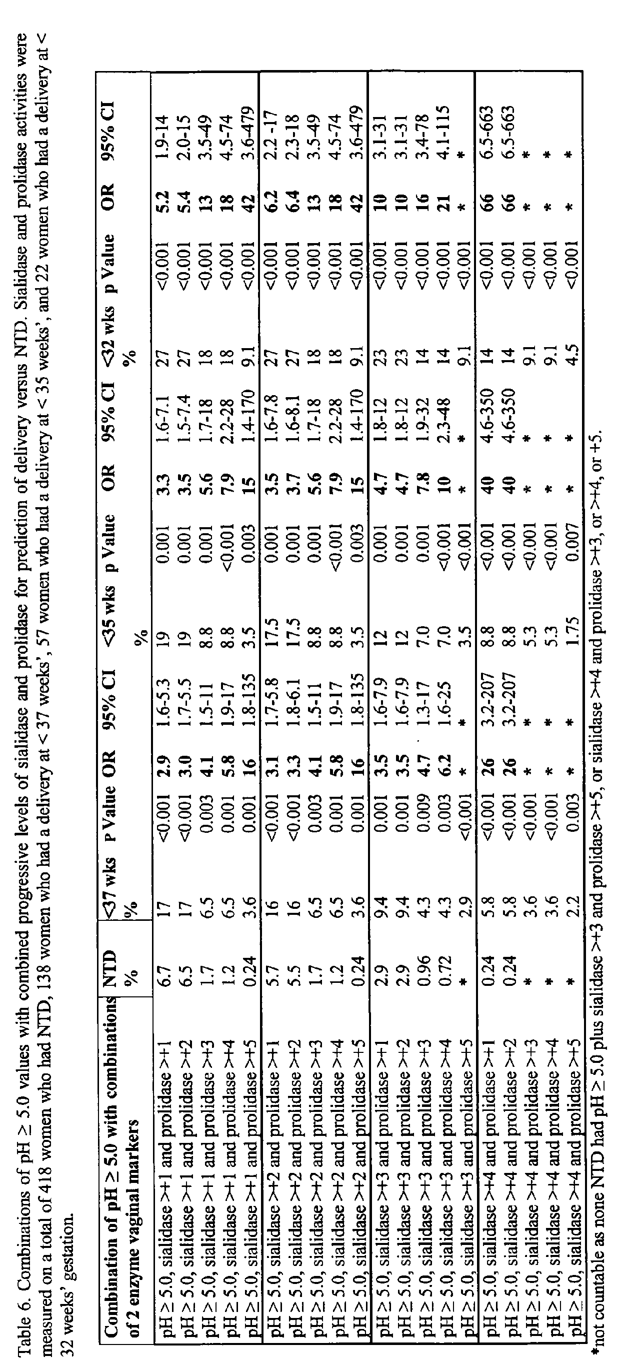

## Claims

1. Method for selecting a particular population of women having a risk of developing obstetric or gynecologic pathologies indicated as OR value equal or higher than 5.5, which value is calculated as the ratio between respectively the percentage of women having no pathologies and those having pathologies, comprising the following steps in order:
a) determination of the levels of sialidase by means of the procedure described in Cauci et al. Am J Obstet Gynecol 1998; 178; 511-5 and/or prolidase activity by means of the procedure described in Cauci et al. J Infect Dis 1998; 178; 1698-706 in samples of body fluid;
b) determination of the pH value of said body fluid samples;
c) selecting the samples having a sialidase value equal or above 5.0 nmol of methoxyphenol and/or a prolidase level equal or above 1500 mOD for prolidase and a pH ≥ 5.0.

2. Method as set forth in claim 1, in which the pH is ≥ 5.0 and ≤ 7.0, preferably ≥ 5.0 and ≤ 6.0, more preferably ≥ 5.0 and ≤ 5.5.

3. Method as set forth in claim 1 or 2, in which after the a) phase a score of said levels of sialidase and/or prolidase activity is determined.

4. Method as set forth in any one of claims 1-3, in which said method is carried out in samples of vaginal fluid.

5. Method as set forth in any one of claims 1-4, in which the obstetric or gynecologic pathologies comprise: low birth weight (LBW), very low birth weight (VLBW) preterm delivery (PTD), early preterm delivery (EPTD), premature rupture of membranes, preterm premature rupture of membranes, intraamniotic infections, spontaneous abortion, endometritis, obstetric surgery infections, post-partum or post-gynecologic surgery infections, pelvic surgery infections, upper genital tract infections which cause infertility, pelvic inflammatory disease (PID), annexitis, cervicitis, sexually transmitted diseases and infections, malignancies of the urogenital tract.

6. Method as set forth in any one of claims 1-5, in which said population of women has the risk of said pathologies at a period of gestation less than 37 weeks, preferably less than 35 weeks, more preferably less than 32 weeks.

7. Method as set forth in any one of claims 1-5, in which said method is carried out in samples of body fluid of pregnant women.

8. Method as set forth in claim 7, in which said method is carried out in samples of body fluid of women in the first or second trimester of gestation.

9. Method as set forth in claim 7, in which said method is carried out in samples of body fluid of women from the sixth to the twenty-fourth full week of gestation.

10. Method as set forth in any one of claims 1-5, in which said method is carried out in samples of body fluid of non-pregnant women.

11. Method as set forth in any one of claims 1-10, in which said OR value is calculated and corrected by a standard factor by the SPSS computer statistic program.

12. Method for selecting a particular population of women having a risk of developing, VLBW, delivery at < 37 weeks' gestation, < 35 weeks' gestation or < 32 weeks' gestation, comprising the following steps in order:
a) determination of the levels of sialidase by means of the procedure described in Cauci et al. Am J Obstet Gynecol 1998; 178; 511-5 and/or prolidase activity by means of the procedure described in Cauci et al. J Infect Dis 1998; 178; 1698-706 in a sample of body fluid;
b) determination of the pH value of said body fluid sample;
c) selecting the samples having a pH ≥ 5.0 and a sialidase value above 0.19 nmol of methoxyphenol and/or a prolidase value above 22 mOD.

13. Method according to claim 12, wherein the step c) comprises selecting the samples having a pH ≥ 5.0, sialidase level of over 2.50 nmol of methoxyphenol or prolidase level of over 1000 mOD.

14. Method according to claim 12, wherein the step c) comprises selecting the samples having a pH ≥ 5.0, a sialidase value above 0.19 nmol or 0.38 nmol or 2.5 nmol of methoxyphenol when it is selected a prolidase value of over 1000 mOD.

15. Method according to claim 12, wherein the step c) comprises selecting the samples having a pH ≥ 5.0, a sialidase value of 0.38 nmol of methoxyphenol and a prolidase value of over 22 mOD or over 44 mOD or over 1000 mOD or over 1500 mOD or over 2000 mOD.

16. Method according to any one of claims 12-15, wherein said risk is indicated as OR value equal or higher than 5.5, which value is calculated preferably by the SPSS computer statistic program.

17. Kit for the determination of women having a risk of developing obstetric or gynecologic pathologies indicated as OR value higher than 5.5, calculated preferably by the SPSS computer statistic program, comprising a sialidase and/or prolidase activity assay in solution that includes a colorless substrate solution in which to inoculate the biologic sample; a developing solution in a container equipped with dispenser; a reference scale to correlate the level of sialidase activity equal or above 0.19 nmol of methoxyphenol and/or the level of prolidase equal or above 22 mOD with the intensity of the developed color; a pH indicator; a reference scale to correlate the pH detected by said indicator with a pH ≥ 5.0 and an illustrative leaflet containing the instructions for the proper use of the kit.

18. Kit according to claim 17, wherein said kit is used with a sample of body fluid of women.

19. Kit according to claim 17 or 18, wherein said body fluid is a vaginal fluid.

20. Kit according to any one of claims 17-19, wherein said pH indicator comprises a revealing paper with a turning interval in the range between 5.0 and 7.0, preferably between 5.0 and 6.0, more preferably between 5.0 and 5.5.

21. Kit according to any one of claims 17-20, wherein said reference scale for the sialidase and/or prolidase activity reports standard values associated with enzyme detecting colors.

22. Kit according to claim 21, wherein said reference scale for pH value associates said turning interval with a particular color intensity of the same color.

23. Kit according to any one of claims 17-22, wherein said illustrative leaflet correlates the enzymatic activity with the pH value in order to evaluate the risk of pathologies as: absent or low (-), medium (+), high (++), very high (+++).

24. Kit according to any one of claims 17-23, including a test on solid support, preferably on reactive strip or platform test, for the determination of the sialidase and/or prolidase activity.

25. Kit according to any one of claims 17-24, comprising as chromogenic or fluorogenic substrate for the determination of sialidase activity a reagent chosen in the group comprising: 2-(3'-methoxyphenyl)-N-acetyl-D-neuraminic acid, 2-O-(o-nitrophenyl)-alfa-D-N-acetyl neuraminic acid, 2'-(4-methylumbelliferyl)-alfa-D-N-acetyl neuraminic acid sodium salt, 5-bromo-4-chloro-3-indolyl-alfa-D-N-acetyl neuraminic acid.

26. Kit according to claim 25, comprising as chromogenic or fluorogenic substrate for the determination of prolidase activity a reagent chosen in the group comprising: L-proline-para-nitroanilide, L-proline-beta-naphthylamide, N-benzyloxycarbonyl-L-prolyl-beta-naphthylamide, N-benzyloxycarbonyl-L-proline-para-nitrophenyl ester, hydroxy-L-prolyl-beta-naphthylamide, L-proline-7-amido-4-methyl-coumarin, L-proline-4-methoxy-beta-naphthylamide.

27. Kit for the determination of women having a risk of developing LBW, VLBW, PTD, delivery at < 37 weeks' gestation, < 35 weeks' gestation or < 32 weeks' gestation, comprising a sialidase and/or prolidase activity assay in solution that includes a colorless substrate solution in which to inoculate the biologic sample; a developing solution in a container equipped with dispenser; a reference scale to correlate the level of sialidase activity equal or above 0.19 nmol of methoxyphenol and/or the level of prolidase equal or above 22 mOD with the intensity of the developed color; a pH indicator; a reference scale to correlate the pH detected by said indicator with a pH ≥ 5.0 and an illustrative leaflet containing the instructions for the proper use of the kit.
